Europäisches Patentamt

European Patent Office

Office européen des brevets

(11) Publication number: **0 245 509 B1**

# EUROPEAN PATENT SPECIFICATION

(45) Date of publication of patent specification: **09.09.92**  (51) Int. Cl.⁵: **G01N  33/544**

(21) Application number: **86906453.5**

(22) Date of filing: **30.10.86**

(86) International application number:
**PCT/JP86/00552**

(87) International publication number:
**WO 87/02780 (07.05.87 87/10)**

(54) **METHOD OF IMMUNOASSAY.**

(30) Priority: **05.11.85 JP 248329/85**

(43) Date of publication of application:
**19.11.87 Bulletin  87/47**

(45) Publication of the grant of the patent:
**09.09.92 Bulletin  92/37**

(84) Designated Contracting States:
**AT BE CH DE FR GB IT LI LU NL SE**

(56) References cited:
| | |
|---|---|
| **JP-A- 5 013 525** | **JP-A- 5 951 938** |
| **JP-A-49 125 517** | **JP-A-58 176 547** |
| **JP-A-60 108 755** | **JP-A-60 142 259** |
| **JP-A-60 155 129** | **JP-B- 5 010 923** |

(73) Proprietor: **KANEBO, LTD.**
**17-4 Sumida 5-chome Sumida-ku**
**Tokyo 131(JP)**

(72) Inventor: **HORIKAWA, Yukio**
**27-12, Shibagaki 1-chome**
**Matsubara-shi Osaka-fu 580(JP)**
Inventor: **IBARAKI, Satoshi**
**6-5-304, Tomobuchicho 1-chome Miyakojima-ku**
**Osaka-shi Osaka-fu 534(JP)**
Inventor: **KANNO, Hiroshi**
**44-11-305, Yamadaminami**
**Suita-shi Osaka-fu 565(JP)**
Inventor: **IWAMOTO, Seiichi**
**14-1, karibadai 1-chome**
**Nishi-ku, Kobe-shi Hyogo-ken 673(JP)**

(74) Representative: **Holmes, Michael John et al**
**Frank B. Dehn & Co. European Patent Attorneys Imperial House 15-19 Kingsway**
**London, WC2B 6UZ,(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 245 509 B1

## Description

Technical Field

This invention relates to an immunoassay method wherein fibroin-entrapped, immobilized antibodies or antigens are repeatedly used in the assay.

Background Art

It has been wide practice to detect or assay certain antigens and antibodies in a sample utilizing a highly specific antigen-antibody reaction as an aid of diagnosis or treatment of certain diseases. Radioimmunoassays and enzyme immunoassays, in particular, find ever-increasing utility as a microassay technique in various clinical tests.

For the sake of accuracy and simplicity, these assays are generally performed by the so-called "solid phase method" in which an antibody (or antigen) specific to an antigen (or antibody) to be assayed is immobilized by chemically binding, adsorbing or entrapping to a suitable insoluble carrier and the resulting immobilized antibody (or antigen) is complexed with the antigen (or antibody) to be assayed.

If the immobilized antibodies or antigens can be used repeatedly in the solid phase immunoassays, such valuable materials may be saved and adapted for use in an automated continuous assay system enabling a great number of samples to be handled easily.

US-A-3970429 ( Updike) and US-A-4009005 describe radioimmunoassay systems which may be suitable for continuous use in repeated cycles where immobilised antibody is regenerated for repeated use by rising with a suitable solvent or eluting solution. In both cases the regeneration systems described comprise the use of organic solvents. In the case of US-A-3970429, the antibody is immobilised in particles of acrylamide gel, whereas in US-A-4009005 the antibody is covalently bound to a solid support.

In order that an immobilized antibody or antigen can be used repeatedly, it is imperative that the antigen-antibody complex formed in the assay may be completely dissociated and the immunoreactivity of the immobilized antibody or antigen may not be lost significantly.

Disclosure of the Invention

As a result of our studies on the aforementioned points, we have found that a fibroin film-entrapped, immobilized antibody or antigen specific to an antigen or antibody to be assayed may be repeatedly used in the immunoassay of said antigen or antibody by complexing said antigen or antibody and a labelled antibody or antigen, determining the amount of said antigen or antibody to be assayed based on the amount of labelled antibody or antigen so complexed, and immersing the immobilized antibody or antigen in a buffer solution having a pH of about 2 to 4 to remove complexed antigens or antibodies from the immobilized antibody or antigen. The immunoreactivity of the immobilized antibody or antigen is not lost significantly by this treatment so that the immobilized antibody or antigen may be used in the same immunoassay repeatedly.

The present invention has been accomplished based on the above findings.

Preferred Embodiment of the Invention

Immobilized antibodies or antigens entrapped in fibroin films used in the present invention may be prepared by the method disclosed in Japanese Patent Publication (Kokai) 60-142259 or 60-155129 (see, Production Examples below). Both publications describe methods for entrapment of antigens or antibodies in fibroin films and how such films may be prepared.

Examples of substances which may be assayed by the method of the present invention include:

(1) polypeptide hormones such as insulin, human chorionic gonadotropin, human placental lactogen, luteinizing hormone and their antibodies;

(2) blood serum proteins such as IgG, IgA, IgM, IgE, alpha-fetoprotein, carcinoembryonic antigen, haptoglobin and their antibodies;

(3) toxins and viruses such as E.coli toxin, cholera toxin, hepatitis virus, rubella virus, influenza virus and their antibodies; and

(4) steroidal hormones and drugs such as estradiol, progesterone, testosterone, phenytoin, procainamide, kanamycin, penicillin, barbiturates and their antibodies.

In the assay of the above-described substances, an immobilized antibody or antigen specific to an antigen or antibody to be assayed is used by entrapping the specific antibody or antigen in a fibroin film.

The assay may be carried out either by the competitive method or the non-competitive method, i.e. the sandwich method.

The antibody or antigen may be labeled with an enzyme, a fluorescent marker or a radioisotope.

Examples of buffer solutions having a pH of about 2 to 4 include glycine-hydrochloric acid buffer and tartaric acid-sodium hydroxide buffer each having a concentration of 0.05 to 0.2 moles/liter. By immersing usually at a temperature of 10°C to 40°C for 3 to 10 minutes, the immobilized antibody or antigen may be easily freed of the antigen or antibody and the labelled antibody or antigen complexed thereto without losing its immunoreactivity so that it may be used in the same immunoassay repeatedly.

At a pH higher than the above range, the dissociation of the antigen-antibody becomes harder to occur, whereas at a pH lower than the above range the immobilized antibody or antigen tends to become denatured undesirably.

The above dissociation reaction may be facilitated further by incorporating 0.5 to 10 weight % of a neutral salt such as sodium chloride, potassium chloride or sodium sulfate to the buffer.

According to the method of the present invention, the immobilized antibodies or antigens may be used repeatedly in various immunoassays as shown in the test results below. Thus, not only the valuable antibodies or antigens may be recycled but also they may be easily adapted for use in an automated continuous assay system for handling a great number of samples easily.

EXPERIMENT 1

Repeated assays of standard alpha-fetoprotein (AFP) using immobilized monoclonal anti-human AFP antibody entrapped in fibroin film

(1) Material:

The fibroin film prepared in hereinafter-described Production Example 1 was cut to a segment of 7 x 12 mm size. The film was bonded at a short edge to one end of a polyester strip having a thickness of 200 $\mu$m (2 mm width x 10 cm length) with a cyanoacrylate adhesive.

(2) Method:

To a test tube having an inner diameter of 10 mm was added 0.5 ml of normal saline containing 0.5 weight % of bovine serum albumin (BSA). The above fibroin film (n=2) was completely immersed in the liquid in the tube, and incubated at room temperature for 1 hour. After sucking off the liquid, 0.5 ml of a solution consisting of 80 ng/ml of standard AFP, 10 weight % of horse serum, peroxidase-labelled monoclonal anti-human AFP antibody (raised in mice but having a recognition site different from that of the immobilized monoclonal antibody, 1.2 $\mu$g/ml, enzyme labelling was carried out by the periodate method described in Journal of Histochemistry and Cytochemistry, 22, 1084 (1974)) and 0.1 weight % of BSA in normal saline was added to the test tube and incubated at 22°C for 1 hour. The film was washed with distilled water three times and transferred to a separate test tube having an inner diameter of 15 mm. Then 0.5 ml of a solution consisting of 19.1 mM o-phenylenediamine and 2.45 mM hydrogen peroxide in citric acid-disodium phosphate buffer, pH 5.0, was added to the tube and placed in the dark at room temperature for 30 minutes. After removing the film, 2 ml of 1N sulfuric acid was added to stop the reaction. Adsorption at 492 nm ($A_{492}$) of the resulting reaction mixture was measured.

The film removed from the tube was immersed in the buffer (a) shown in Table I for 5 minutes, washed with water and used in a blank test by repeating the above procedure without adding standard AFP. This cycle was repeated successively.

Next, similar tests were carried out using the buffer (b) shown in Table I as a dissociating solution.

(3) Results:

The results obtained are shown in Table I.

Table I

| Mean $A_{492}$ (n = 2) | | | |
|---|---|---|---|
| Times of Assays | AFP Concentration (ng/ml) | Dissociating Solution Consisting of 0.1 M glycine-HCl | |
| | | (a) At pH 2.2 containing 1% NaCl | (b) At pH 2.9 containing 5% KCl |
| 1 | 80 | 1.119 | 1.077 |
| 2 | 0 | 0.025 | 0.027 |
| 3 | 80 | 1.017 | 1.155 |
| 4 | 0 | 0.029 | 0.034 |
| 5 | 80 | 1.083 | 1.172 |
| 6 | 0 | 0.024 | 0.036 |
| 7 | 80 | 1.152 | 1.106 |
| 8 | 0 | 0.033 | 0.029 |
| 9 | 80 | 1.143 | 1.128 |
| 10 | 0 | 0.031 | 0.030 |
| 11 | 80 | 1.054 | 1.039 |
| 12 | 0 | 0.027 | 0.035 |
| 13 | 80 | 1.121 | 1.014 |
| 14 | 0 | 0.035 | 0.033 |
| 15 | 80 | 1.027 | 1.185 |
| 16 | 0 | 0.033 | 0.038 |
| 17 | 80 | 1.035 | 1.065 |
| 18 | 0 | 0.028 | 0.031 |
| 19 | 80 | 1.077 | 1.118 |
| 20 | 0 | 0.032 | 0.035 |

EXPERIMENT 2

Repeated assay of standard human chorionic gonadotropin (hCG) using immobilized monoclonal anti-hCG antibody entrapped in fibroin film

(1) Material:

The fibroin film prepared in hereinafter-described Production Example 2 was cut to a segment of 1.2 x 0.7 cm size and bonded to a polyester strip as in Experiment 1.

(2) Method:

The above films (n = 10 in each concentration) were immersed in 0.5 ml of a series of solutions of 0, 10, 30, 100 and 300 mIU/ml of standard hCG in normal saline containing 0.1 weight % of BSA, respectively, and incubated at 25°C for 1 hour. Thereafter the respective films were washed with water and then re-immersed in 0.5 ml of a solution consisting of horseradish peroxidase-labeled anti-hCG antibody (1.0 $\mu$g/ml, rabbit IgG, labeled by the same method as in Experiment 1) and 0.1 weight % of BSA in normal saline, and incubated at 25°C for 1 hour. After washing with distilled water to remove free portions of the labeled antibody, the films were subjected to the enzyme-developing reaction as in Experiment 1. $A_{492}$ of the resulting reaction mixtures were measured respectively.

The films removed from respective tubes were immersed in the buffer (a) shown in Table II for 5 minutes, washed with water and reused in the next run repeatedly. Each film was used at random for the series of solutions of standard hCG.

Next, similar tests were carried out using the buffer (c) shown in Table II as a dissociating solution.

(3) Results:

The results obtained are shown in Table II.

TABLE II

| hCG concentration (mIU/ml) | Mean $A_{492}$ (n = 10) and Coefficient of Variation (%) | |
| --- | --- | --- |
| | Dissociating solution consisting of 0.1 M glycine-HCl | |
| | (a) At pH 2.2 containing 1% NaCl | (c) At pH 2.9 containing 5% NaCl |
| 0 | 0.021 (9.1) | 0.030 (12.3) |
| 10 | 0.090 (7.6) | 0.093 ( 8.4) |
| 30 | 0.254 (7.8) | 0.285 (10.7) |
| 100 | 0.567 (5.3) | 0.541 ( 6.1) |
| 300 | 0.825 (6.2) | 0.857 ( 7.5) |

The invention will be described in more detail by making reference to the following Production Examples and Example.

PRODUCTION EXAMPLE 1

Preparation of immobilized monoclonal anti-human AFP antibody entrapped in fibroin film (see, Japanese Patent Publication (Kokai) 60-155129)

(1) Preparation of aqueous solution of fibroin:

100 g of silk fiber was soaked in 5 liters of a 1.0 weight % aqueous solution of marseilles soap and degummed at 80°C for three hours. After washing with water, the fiber was soaked in 5 liters of a 0.5 weight % aqueous solution of marseilles soap and degummed at 80°C for three hours to obtain 72 g of fibroin material substantially free from sericin etc.

150 g of calcium chloride was dissolved in a mixture of 100 g of water and 80 g of ethanol in a kneader and the solution was heated at 75°C. 70 g of the above fibroin material was dissolved in the above solution with stirring for 1 hour and then diluted with 180 g of hot water (75°C). After cooling, the resulting fibroin solution was dialyzed in a hollow fiber dialyzer against tap water to give 1200 ml of a 5.7 weight % solution of fibroin. The residual calcium chloride concentration was 0.08 weight %.

(2) Preparation of immobilized monoclonal anti-human AFP antibody entrapped in fibroin film:

Monoclonal anti-human AFP antibody (raised in mice) dissolved in normal saline at a concentration of 250 $\mu$m/ml was applied on a glass plate surrounded by a square frame in an amount of 10 $\mu$g of the antibody/$cm^2$ and dried at 15°C for 3 hours. After adding an amount of glycerine corresponding to 30 weight % of fibroin, the fibroin solution prepared in step (1) was cast on the antibody layer and dried at 20°C for 10 hours to give a fibroin film entrapping monoclonal anti-human AFP antibody having a thickness of 60 $\mu$m.

PRODUCTION EXAMPLE 2

Preparation of immobilized monoclonal anti-hCG antibody entrapped in fibroin film (see, Japanese Patent Publication (Kokai) 60-155129)

Monoclonal anti-hCG antibody (raised in mice) dissolved in normal saline at a concentration of 250 μg/ml was applied on a glass plate surrounded by a square frame in an amount of 10 μg of the antibody/cm² and dried at 20°C for 3 hours.

The fibroin solution produced in Production Example 1 was concentrated to 15.7 weight % and mixed with an amount of glycerine corresponding to 30 weight % of fibroin.

Then the mixture was cast on the antibody layer and dried at 20°C for 8 hours to give a fibroin film entrapping monoclonal anti-hCG antibody having a thickness of 90 μm.

EXAMPLE

Repeated immunoassays of AFP in human sera

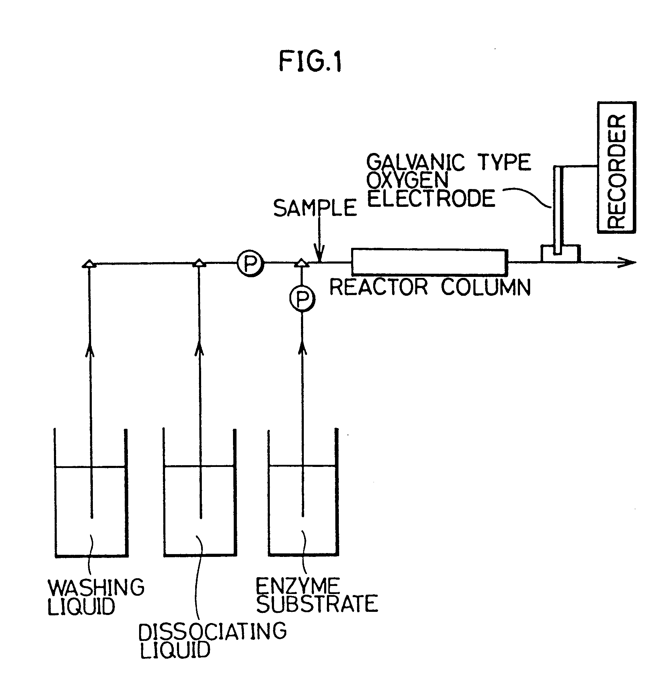

The fibroin film entrapping monoclonal anti-human AFP antibody prepared in Production Example 1 was cut to a strip of 0.25 x 10 cm size. A flow type assay system shown in Fig. 1 of the accompanying drawings was assembled by placing the film strip in a glass tube having an inner diameter of 0.3 cm and a length of 11 cm to form a reactor column.

(1) Preparation of standard curve:

The reactor column was filled with distilled water. Then 0.7 ml of a series of solutions of 0, 1.25, 2.5, 5, 10, 20, and 40 ng/ml of standard AFP in normal saline containing 0.8 μg/ml of catalase-labelled anti-human AFP antibody as described below and 0.1 weight % of BSA was introduced into the reactor column and incubated for 20 minutes. The reactor column was washed by passing distilled water at a flow rate of 20 ml/minute for 1 minute. Then 0.7 ml of 0.1 M phosphate buffer, pH 7.0, containing 17.6 mM of hydrogen peroxide was introduced into the reactor column and allowed to react for 5 minutes. The reaction mixture was then displaced to a galvanic type oxygen electrode cell (Model AN, manufactured by Oriental Electric Co., Ltd.) by introducing distilled water at a flow rate of 0.5 ml/minute. Increase in potential was determined from the readings of electric current which were proportional to the oxygen concentration in the reaction mixture.

After the completion of the above procedure, the film was freed of bound antibody and antigen by passing 0.1 M glycine-HCl buffer (pH 2.5, containing 2% NaCl) through the reactor column at a flow rate of 0.5 ml/minute for 3 minutes. The column was then washed with distilled water at a flow rate of 20 ml/minute for 10 seconds.

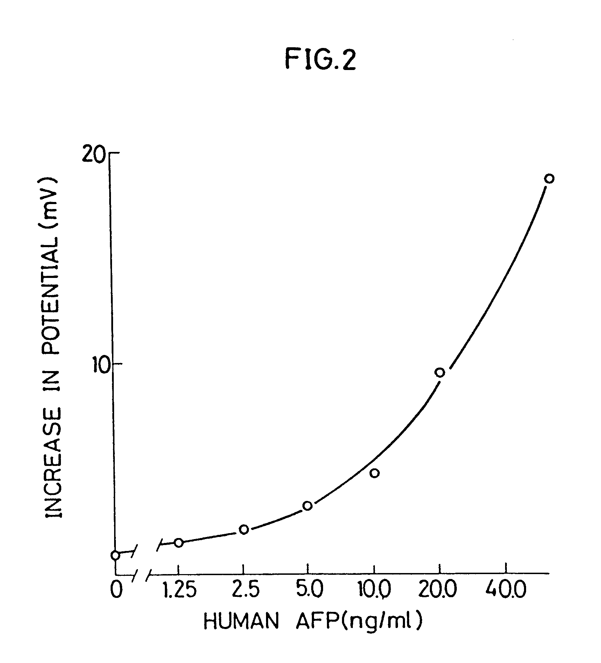

The above procedures were repeated twice for each concentration of standard AFP. A standard curve as shown in Fig. 2 was obtained.

All procedures are performed at room temperature.

The catalase-labelled anti-human AFP antibody was produced as follows:

1 mg of the above antibody and 3 mg of catalase (sigma, bovine liver origin) were dissolved in 1 ml of 0.1 M phosphate buffer, pH 8.0. 10 μl of 10 weight % aqueous solution of glutaraldehyde was added to the solution. The mixture was allowed to react at 15°C for 3 hours. Then 2 mg of sodium borohydride was added. After allowing to react for 30 minutes, the reaction mixture was dialyzed in a dialysis membrane tube against 0.1 M phosphate buffer, pH 7.0, for 4 hours. This reaction mixture was purified using a TSK gel G-3000SW column (registered Trade Mark of TOKYO SODA MFG., Inc.) and a fraction containing the desired labelled antibody was collected.

(2) Repeated immunoassay of AFP in human sera:

A series of samples containing 0, 30, 50, 150 and 300 ng of standard AFP, respectively, was prepared by adding AFP into 1 ml of human serum from healthy adults (known to contain less than 5 ng/ml on radioimmunoassay). Each sample liquid was diluted 10 times with a 0.1 weight % solution of BSA in normal saline containing 0.89 μg/ml of the above catalase-labelled antibody.

Using the above reactor column, the procedures of part (1) were repeated twice for each sample whereupon the results shown in Table III were obtained.

Table III

| AFP added (ng/ml) | Mean AFP found (n = 2) (ng/ml) |
|---|---|
| 0 | <12.5 |
| 30 | 34 |
| 50 | 57 |
| 80 | 83 |
| 150 | 165 |
| 300 | 290 |

As shown in Table III, it was possible to assay AFP in human sera repeatedly.

**Claims**

1. An immunoassay method including the step of complexing an antibody or antigen immobilized by entrapment in fibroin film and specific to an antigen or antibody to be assayed with said antigen or antibody to be assayed, characterised in that after completion of the use in the assay of said immobilized antibody or antigen the latter is contacted with a buffer solution having a pH of about 2 to 4 to remove complexed antigens or antibodies therefrom by dissociation, so as to permit the immobilized antibody or antigen to be used in a further such assay.

2. A method as claimed in claim 1 for assaying an antigen wherein a fibroin film entrapped, immobilized antibody to said antigen is employed.

3. A method as claimed in claim 1 or claim 2 wherein said immobilized antibody or antigen is contacted with said buffer solution at a temperature of 10°C to 40°C.

4. A method as claimed in any one of claims 1 to 3 wherein said immobilized antibody or antigen is contacted with said buffer solution for 3 to 10 minutes.

5. A method as claimed in any one of claims 1 to 4 wherein said buffer solution is a glycine-HCl buffer or a tartaric acid-sodium hydroxide buffer having a concentration of 0.05 to 0.02M.

6. A method as claimed in any one of claims 1 to 5 wherein said buffer solution includes 0.5 to 10 weight % of a salt selected from sodium chloride, potassium chloride and sodium sulphate.

7. A method as claimed in claim 6 wherein said buffer solution is 0.1M glycine-HCl containing 1-5 weight % of NaCl or KCl at pH 2.2-2.9.

8. A method as claimed in any one of claims 2 to 7 wherein an antigen to be assayed is further complexed with a labelled antibody.

9. A method as claimed in claim 8 comprising the steps of:
   (a) incubating a fibroin film having entrapped, immobilized antibody specific to the antigen to be assayed with said antigen and the labelled antibody in a flow-type reaction vessel;
   (b) removing from said vessel the liquid phase of the complexing mixture and measuring the uncomplexed label;
   (c) passing through the reaction vessel at a flow rate of 0.5ml/minute for 3 minutes 0.1M glycine-HCl containing 2 weight % of Nacl at pH 2.5; and
   (d) washing the fibroin film with distilled water.

**Patentansprüche**

1. Immuntestverfahren, wobei man einen Antikörper oder ein Antigen, immobilisiert durch Einschluß in einen Fibroinfilm und spezifisch für ein Antigen oder einen Antikörper,das oder der zu testen ist, mit dem zu testenden Antigen oder Antikörper komplexiert,
**dadurch gekennzeichnet**, daß man
nach beendeter Verwendung des immobilisierten Antikörpers oder des immobilisierten Antigens im Test diesen oder dieses mit einer Pufferlösung in Kontakt bringt, die einen pH von etwa 2 bis 4 aufweist, um komplexierte Antigene oder Antikörper davon durch Dissoziation zu entfernen, so daß der immobilisierte Antikörper oder das immobilisierte Antigen für einen weiteren solchen Test verwendet werden kann.

2. Verfahren nach Anspruch 1 zur Bestimmung eines Antigens, wobei man einen in einen Fibroinfilm eingeschlossenen, immobilisierten Antikörper gegen das Antigen anwendet.

3. Verfahren nach Anspruch 1 oder Anspruch 2, worin der immobilisierte Antikörper oder das immobilisierte Antigen mit der Pufferlösung bei einer Temperatur von 10°C bis 40°C in Kontakt gebracht wird.

4. Verfahren nach einem der Ansprüche 1 bis 3, worin der immobilisierte Antikörper oder das immobilisierte Antigen mit der Pufferlösung 3 bis 10 Minuten in Kontakt gebracht wird.

5. Verfahren nach einem der Ansprüche 1 bis 4, worin die Pufferlösung ein Glycin-HCl-Puffer oder ein Weinsäure-Natriumhydroxid-Puffer mit einer Konzentration von 0,05 bis 0,02M ist.

6. Verfahren nach einem der Ansprüche 1 bis 5, worin die Pufferlösung 0,5 - 10 Gew.-% eines Salzes enthält, ausgewählt unter Natriumchlorid, Kaliumchlorid und Natriumsulfat.

7. Verfahren nach Anspruch 6, worin die Pufferlösung 0,1M Glycin-HCl ist, und 1 - 5 Gew.-% NaCl oder KCl bei pH 2,2 bis 2,9 enthält.

8. Verfahren nach einem der Ansprüche 2 bis 7, worin das zu testende Antigen zusätzlich mit einem markierten Antikörper komplexiert wird.

9. Verfahren nach Anspruch 8, wobei man:
(a) einen Fibroinfilm mit einem eingeschlossenen, immobilisierten Antikörper, der für das zu testende Antigen spezifisch ist, mit dem Antigen und dem markierten Antikörper in einem Durchflußreaktionsgefäß inkubiert;
(b) aus dem Gefäß die Flüssigphase des Komplexierungsgemisches entfernt und den nicht-komplexierten Label bestimmt;
(c) durch das Reaktionsgefäß mit einer Flußgeschwindigkeit von 0,5 ml/min 0,1M Glycin-HCl mit einem Gehalt von 2 Gew.-% NaCl 3 Minuten bei pH 2,5 durchleitet; und
(d) den Fibroinfilm mit destilliertem Wasser wäscht.

**Revendications**

1. Méthode de dosage immunologique comprenant le stade de complexation d'un anticorps ou d'un antigène immobilisé par piégeage dans un film de fibroïne et spécifique d'un antigène ou d'un anticorps à doser avec cet antigène ou anticorps à doser, caractérisé en ce que lorsqu'on a fini d'utiliser dans le dosage cet anticorps ou antigène immobilisé, on met en contact ce dernier avec une solution tampon ayant un pH d'environ 2 à 4 pour en éliminer les antigènes ou anticorps complexés par dissociation, de façon à permettre l'utilisation de l'anticorps ou antigène immobilisé dans un dosage ultérieur.

2. Méthode selon la revendication 1 pour doser un antigène dans laquelle on utilise un anticorps pour cet antigène immobilisé, piégé dans un film de fibroïne.

3. Méthode selon les revendications 1 ou 2, dans laquelle cet anticorps ou antigène immobilisé est mis en contact avec cette solution tampon à une température de 10°C à 40°C.

4. Méthode selon l'une quelconque des revendications 1 à 3, dans laquelle cet anticorps ou antigène immobilisé est mis en contact avec cette solution tampon pendant 3 à 10 minutes.

**5.** Méthode selon l'une quelconque des revendications 1 à 4, dans laquelle cette solution tampon est un tampon glycine-HCl ou un tampon acide tartrique-hydroxyde de sodium ayant une concentration de 0,05 à 0,02M.

**6.** Méthode selon l'une quelconque des revendications 1 à 5, dans laquelle cette solution tampon contient 0,5 à 10% en poids d'un sel choisi parmi le chlorure de sodium, le chlorure de potassium et le sulfate de sodium.

**7.** Méthode selon la revendication 6, dans laquelle cette solution tampon est du chlorhydrate de glycine 0,1M contenant 1 à 5% en poids de NaCl ou KCl à un pH de 2,2-2,9.

**8.** Méthode selon l'une quelconque des revendications 2 à 7, dans laquelle un antigène à doser est en outre complexé avec un anticorps marqué.

**9.** Méthode selon la revendication 8, comprenant les stades consistant à:
(a) faire incuber un film de fibroïne dans lequel est piégé un anticorps immobilisé spécifique de l'antigène à doser avec cet antigène et l'anticorps marqué dans un récipient réactionnel du type à écoulement;
(b) éliminer de ce récipient la phase liquide du mélange complexant et mesurer le marquage non complexé;
(c) faire passer à travers le récipient réactionnel, avec un débit de 0,5 ml/minute, pendant 3 minutes, du chlorhydrate de glycine 0,1M Contenant 2% en poids de NaCl à pH 2,5; et
(d) laver le film de fibroïne à l'eau distillée.

## FIG.1

FIG.2